# EUROPEAN PATENT SPECIFICATION

(11) **EP 1 768 969 B1**
(45) Date of publication and mention of the grant of the patent: **17.03.2010**
(21) Application number: 05773805.6
(22) Date of filing: 20.07.2005
(51) Int. Cl.: C07D 307/88

(54) **CRYSTALLINE MYCOPHENOLATE SODIUM**
KRISTALLINES MYCOPHENOLAT-NATRIUM
MYCOPHENOLATE DE SODIUM CRISTALLIN

(30) Priority: 29.11.2004 US 631849 P; 20.07.2004 US 589909 P
(43) Date of publication of application: 04.04.2007
(73) Proprietor: Teva Gyógyszergyár Zártköruen Muködo Részvenytarsaság, 4042 Debrecen (HU)
(72) Inventor: MOLNAR, Sandor, H-4031 Debrecen (HU); SZABO, Csaba, H-4031 Debrecen (HU); TAMAS, Tivadar, H-4034 Debrecen (HU); HAJKO, Janos, 4028 Debrecen (HU); KOVACSNE-MEZEI; Adrienne, 4032 Debrecen (HU); ARONHIME, Judith, 76217 Rehovot (IL)
(74) Representative: Wong, Kit Yee
(86) International application number: PCT/US2005/025816
(87) International publication number: WO 2006/012385

(56) References cited:
- WO-A-97/38689
- WO-A-2004/064806
- WO-A-2004/087174
- US-A- 5 543 408
- RIHS G ET AL: "SODIUM MYCOPHENOLATE" ACTA CRYSTALLOGRAPHICA SECTION C. CRYSTAL STRUCTURE COMMUNICATIONS, MUNKSGAARD, COPENHAGEN, DK, vol. C56, no. 7, April 2000 (2000-04), pages 432-433, XP008052367 ISSN: 0108-2701 cited in the application
- MAKARA G M ET AL: "Nuclear Magnetic Resonance and Molecular Modeling Study on Mycophenolic Acid: Implications for Binding to Inosine Monophosphate Dehydrogenase" JOURNAL OF MEDICINAL CHEMISTRY, AMERICAN CHEMICAL SOCIETY. WASHINGTON, US, vol. 39, 1996, pages 1236-1242, XP002360234 ISSN: 0022-2623 cited in the application
- DATABASE WPI Section Ch, Week 197018 Derwent Publications Ltd., London, GB; Class B02, AN 1970-31237R XP002360270 -& ZA 6 804 959 A (LILLY & CO ELI) 1 January 1970 (1970-01-01) cited in the application
- CAIRA M R: "CRYSTALLINE POLYMORPHISM OF ORGANIC COMPOUNDS" TOPICS IN CURRENT CHEMISTRY, SPRINGER, BERLIN, DE, vol. 198, 1998, pages 163-208, XP001156954 ISSN: 0340-1022
- GUILLORY J K: "GENERATION OF POLYMORPHS, HYDRATES, SOLVATES, AND AMORPHOUS SOLIDS" POLYMORPHISM IN PHARMACEUTICAL SOLIDS, XX, XX, 1999, pages I-II,183, XP002350313
- CRAIG D Q M ET AL: "The relevance of the amorphous state to pharmaceutical dosage forms: Glassy drugs and freeze dried systems" INTERNATIONAL JOURNAL OF PHARMACEUTICS, AMSTERDAM, NL, vol. 179, no. 2, 15 March 1999 (1999-03-15), pages 179-207, XP002274233 ISSN: 0378-5173

## Description

### CROSS-REFERENCE TO RELATED APPLICATIONS

This application claims the benefits of U.S. Provisional Patent Application Nos. 60/589,909, filed July 20, 2004, and 60/631,849 filed November 29, 2004.

### FIELD OF THE INVENTION

The present invention relates to the solid state chemistry of mycophenolate sodium.

### BACKGROUND OF THE INVENTION

At the end of 1960's, Eli Lilly disclosed the inhibiting effect of mycophenolate sodium salt (MPS) on the growth of malignant tumor cells in warm-blooded mammals. Nowadays Novartis has introduced an enteric-coated formulation of mycophenolate sodium, referred to as Myfortic^{®}. Mycophenolic acid can be formed either as mono- or disodium salt. South African patent No. 6804959 describes the preparation of mono- and disodium mycophenolate. Monosodium mycophenolate can be isolated after reaction of one molar equivalent of sodium methoxide with mycophenolic acid in a mixture of methanol and chloroform by precipitation with *n*-pentane. Preparation of the corresponding disodium salt is also described. In this case two molar equivalents of sodium methoxide were added to a solution of mycophenolic acid in 2:1 benzene-chloroform mixture. The evaporated material was crystallized from aqueous acetone.

The synthetic route of WO 97/38689 is identical to the one described in South African patent No. 6804959. The compound may be obtained in crystalline form by recrystallization from acetone/ethanol if necessary with water (m.p.189-191°C).

The present invention relates to the solid state physical properties of mycophenolate sodium. These properties may be influenced by controlling the conditions under which mycophenolate sodium is obtained in solid form. Solid state physical properties include, for example, the flowability of the milled solid. Flowability affects the ease with which the material is handled during processing into a pharmaceutical product. When particles of the powdered compound do not flow past each other easily, a formulation specialist must take that fact into account in developing a tablet or capsule formulation, which may necessitate the use of glidants such as colloidal silicon dioxide, talc, starch or tribasic calcium phosphate.

Another important solid state property of a pharmaceutical compound is its rate of dissolution in aqueous fluid. The rate of dissolution of an active ingredient in a patient's stomach fluid may have therapeutic consequences since it imposes an upper limit on the rate at which an orally-administered active ingredient may reach the patient's bloodstream. The rate of dissolution is also a consideration in formulating syrups, elixirs and other liquid medicaments. The solid state form of a compound may also affect its behavior on compaction and its storage stability.

These practical physical characteristics are influenced by the conformation and orientation of molecules in the unit cell, which defines a particular polymorphic Form of a substance. The polymorphic form may give rise to thermal behavior different from that of the amorphous material or another polymorphic form. Thermal behavior is measured in the laboratory by such techniques as capillary melting point, thermogravimetric analysis (TGA) and differential scanning calorimetry (DSC) and may be used to distinguish some polymorphic forms from others. A particular polymorphic form may also give rise to distinct spectroscopic properties that may be detectable by powder X-ray crystallography, solid state C NMR spectrometry and infrared spectrometry.

WO2004/020426 discloses preparation of sodium mycophenolate by reacting mycophenolic acid or its ammonium or dibenzyl-amine salt with a sodium salt of C₂ to C₁₀ carboxylic acid. Mycophenolic acid is converted to its ammonium salt by reacting with ammonia. This compound is reacted with sodium acetate to obtain the sodium salt of mycophenolic acid.

WO 2004/064806 discloses additional polymorphic forms of mycophenolate sodium and acid.

### Monosodium Salt

South African patent No. 68/4,959 provides an example for preparing monosodium mycophenolate salt (Example 3). Sodium methylate in anhydrous methanol was added to mycophenolic acid in chloroform, then the monosodium salt was precipitated by adding n-pentane and collected by filtration and vacuum dried.

Acta Chrtystallographica Sect. C, (2000), C56, 432-433 describes another process for producing monosodium mycophenolate. A methanolic solution of the commercially available mycophenolic acid was treated with one equivalent of sodium methanolate. After stirring for 1 hour at room temperature, the solvent was evaporated to dryness in vacuum. The melting point of the product was 463 K (190°C). Single crystals were grown by evaporation and cooling of a water/ethyl acetate solution from about 323K to room temperature. The crystal structure of the produced sodium mycophenolate measured by single crystal diffractometer is also described.

Based on the given crystal parameters, the calculated powder diffractogram done by the inventors of the present invention show that the crystal form obtained is the crystal form denominated Form M2. Form M2 is an anhydrous form. Form M2 is characterized by a powder XRD pattern with peaks at 5.3, 8.0, 9.8, 10.7, and 21.9 ±0.2 degrees 2 theta (Fig. 3) and FTIR peaks at 1719, 1571, 1317, 1266, 1134 and 927 cm-1 (Fig. 4). Form M2 may be further characterized by XRD peaks at 13.6 and 19.0 ±0.2 degrees 2 theta. Form M2 may be further characterized by IR peaks at 1194, 1108, 1075, 1034, 971. 875, 826, 794 and 722 cm-1. Form M2 may be further characterized by a DSC curve (Fig. 9).

PCT 97/38689 describes sodium mycophenolate salts as known from South African Patent. It also describes the process for obtaining monosodium salt in crystalline form by recrystallization from acetone/ethanol if necessary with water. The melting point provided is 189-191°C.

J. Med. Chem. (1996), 39, 1236-1242 describes treating a solution of mycophenolic acid in ethanol with equimolar sodium ethylate at room temperature and stirring for 30 minutes. The solvent was evaporated in vacuum.

J. Pharm. Sciences (1970), 59(8), 1157-1159 asserts that monosodium mycophenolate may be formed by adjusting the slurry of mycophenolic acid to pH 7-8 with sodium hydroxide. No physical data is provided.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

### SUTMMARY OF THE INVENTION

The invention encompasses a crystalline form of monosodium mycophenolate denominated form M5.

The invention also encompasses pharmaceutical compositions comprising the mycophenolate form of the invention.

The present invention provides for anhydrous mycophenolate sodium. The anhydrous mycophenolate sodium of this invention may be further characterized by having not more than 1% weight loss. Mycophenolate sodium Form M5 discussed below is anhydrous mycophenolate sodium.

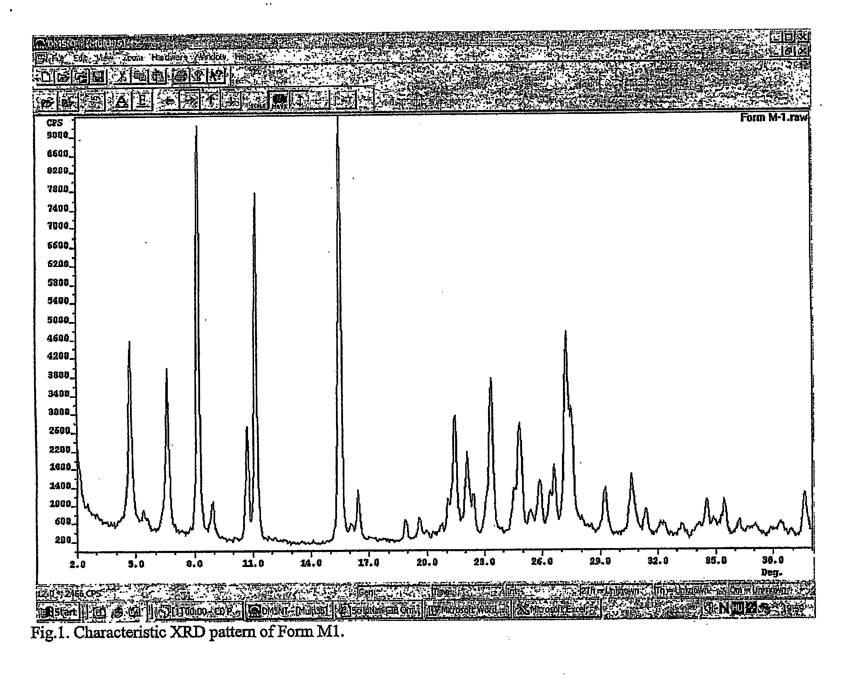

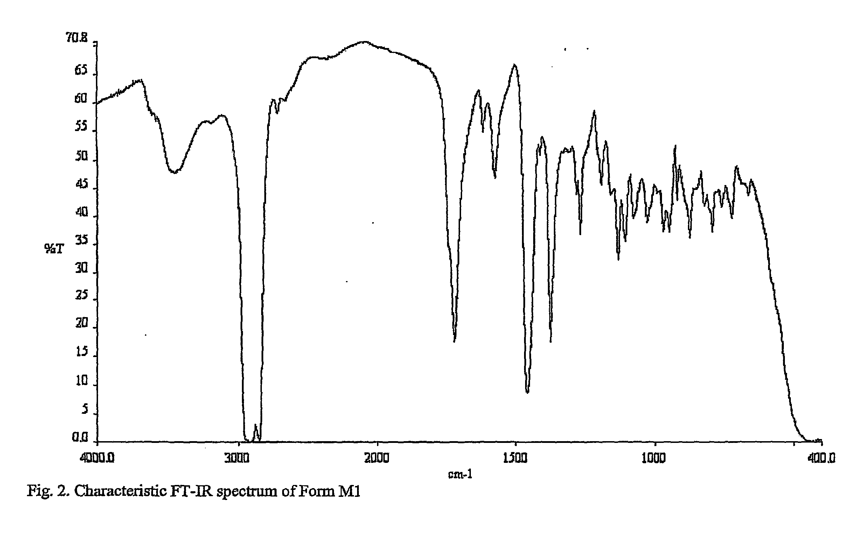

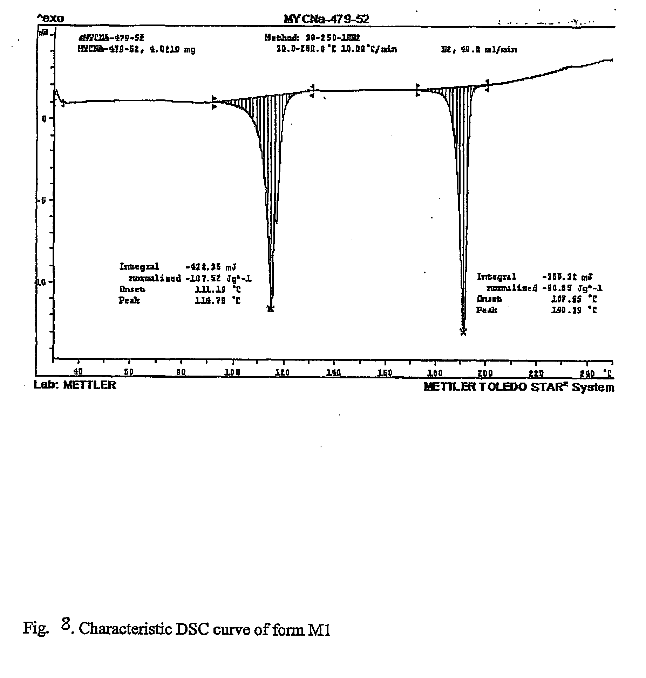

Crystalline mycophenolate sodium Form M1 is characterized by a powder XRD pattern with peaks at 4.7, 6.6, 11.2 and 15.6 ± 0.2 degrees 2 theta (Fig. 1). Form M1 may be further characterized by XRD peaks at 8.2, 21.5 and 23.4 ± 0.2 degrees 2 theta. Form M1 may also be characterized by FTIR peaks at 3450, 1723, 1619, 1578, 1268 and 1132 cm-1 (Fig. 2). In addition, Form M1 may be characterized by DSC peaks as shown in Fig. 8.

One process for preparing crystalline mycophenolate sodium M1 comprises preparing a solution of mycophenolic acid in a C₁-C₄ alcohol; combining a base and a source of sodium with the solution to form a mixture; cooling the mixture to precipitate the crystalline form and recovering the crystalline form before transition to another crystalline form. Stirring is preferably carried out for about 1 to about 5 hours.

Another process for preparing crystalline mycophenolate sodium M1 comprises dissolving sodium mycophenolate in water; crystallizing the crystalline form; and recovering the crystalline form.

Another process for preparing crystalline mycophenolate sodium M1 comprises preparing a solution of sodium mycophenolate in ethanol; crystallizing the crystalline form from the solution; and recovering the crystalline form.

Another process for preparing crystalline mycophenolate sodium M1 comprises contacting crystalline mycophenolate sodium with water vapor.

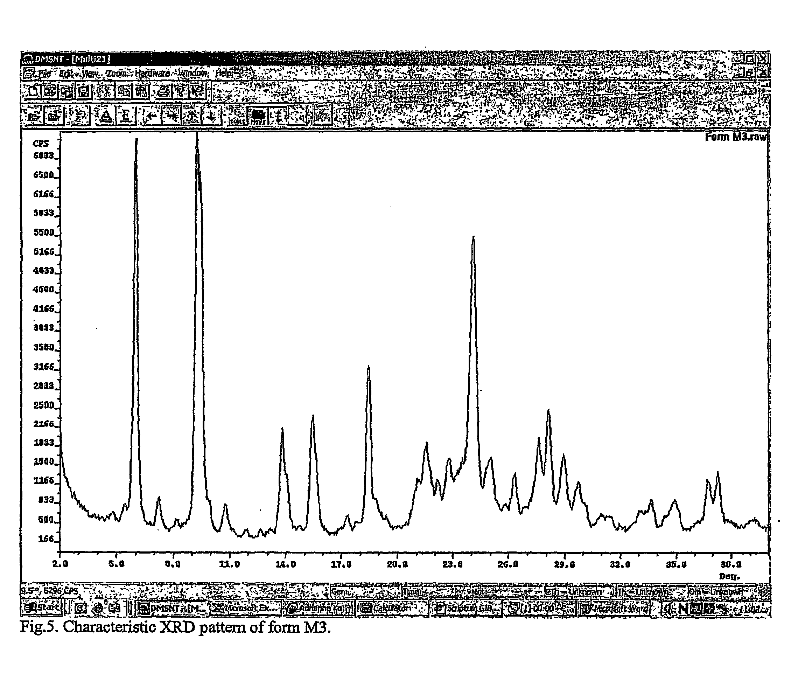

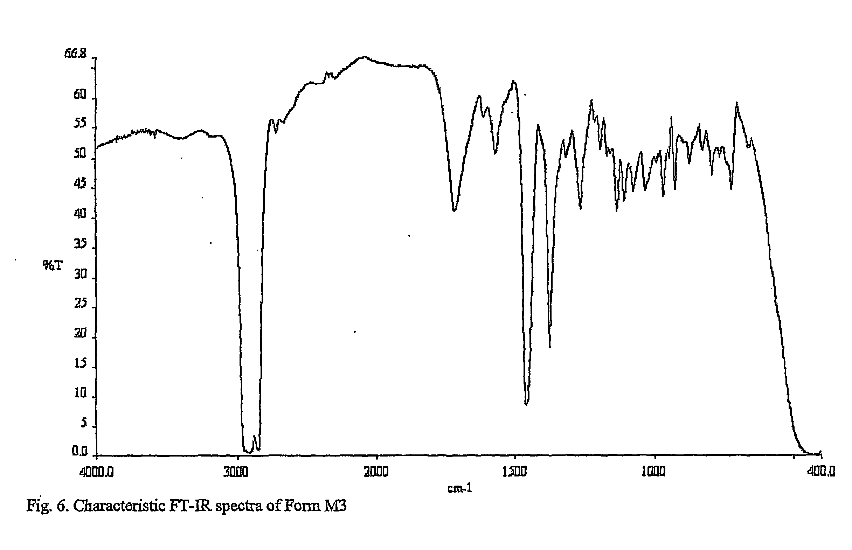

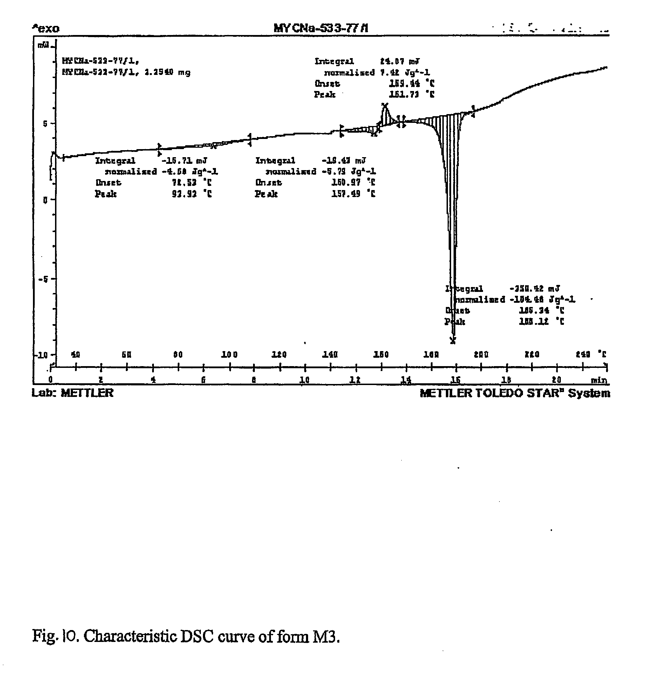

Crystalline mycophenolate sodium Form M3 is characterized by a powder XRD pattern with peaks at 6.0, 9.3, 15.5, and 18.4 ±0.2 degrees 2 theta (Fig. 5) and FTIR peaks at 3414, 1713, 1618, 1567, 1264 and 1134 cm-1 (Fig. 6). Form M3 may be further characterized by a powder XRD pattern with peaks at 7.2, 10.8, 13.8, and 24.0 ±0.2 degrees 2 theta. In addition, Form M3 may be characterized by DSC peaks as shown in Fig. 10.

The process for preparing crystalline mycophenolate sodium M3 comprises the steps of: preparing a solution of mycophenolic acid in a C₁ to C₄ alcohol, preferably methanol; combining a base and a source of sodium with the solution to form a mixture; cooling the mixture to crystallize the crystalline form and recovering the crystalline form. The crystalline form is preferably dried.

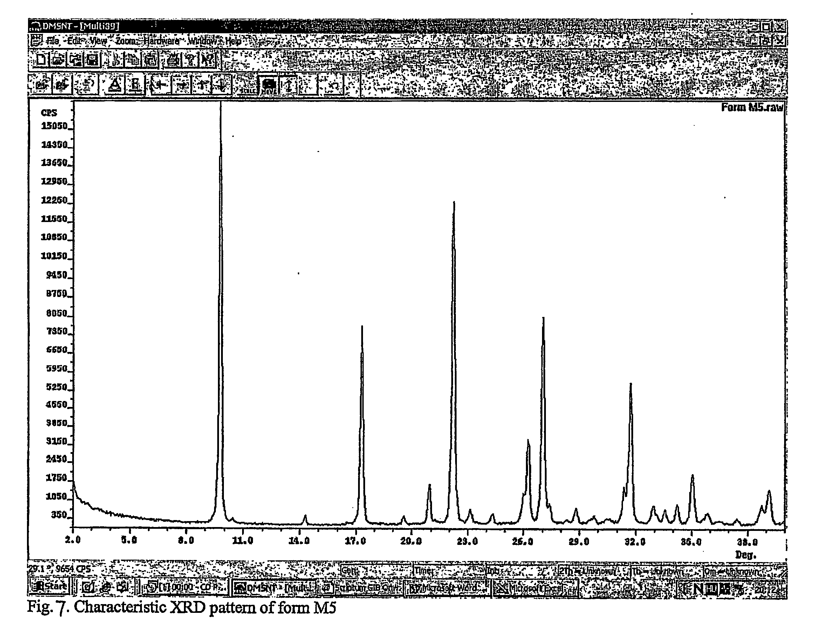

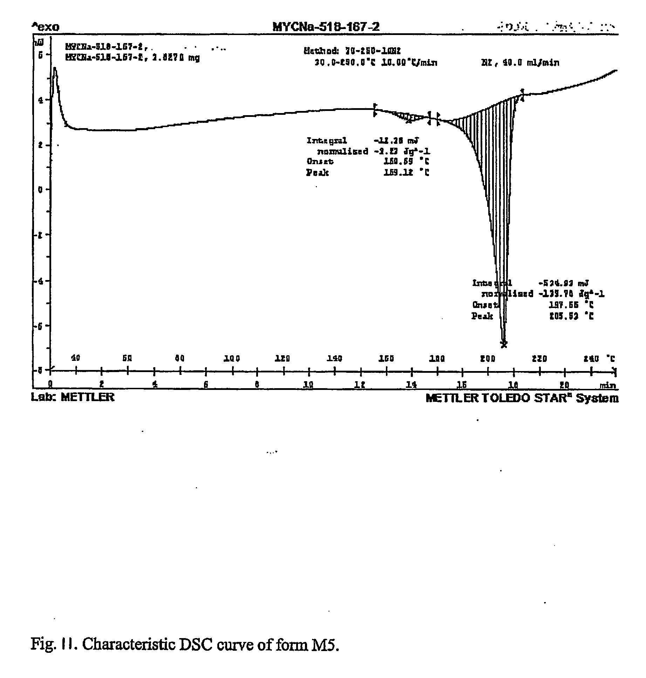

The present invention is a crystalline mycophenolate sodium, denominated Form M5,
characterized by a powder XRD pattern with peaks at 9.8, 17.4, 22.2, 27.1, and 31.7 ±0.2 degrees 2 theta (Fig. 7). Form M5 may be further characterized by XRD peaks at 21.0, 26.3 and 31.4±0.2 degrees 2 theta. In addition, Form M5 may be characterized by DSC peaks as shown in Fig. 11.

The process for preparing crystalline mycophenolate sodium M5 comprises the steps of: preparing a mixture of sodium mycophenolate and 1,4-dioxane; crystallizing the crystalline form from the mixture; and recovering the crystalline form.

The present invention encompasses pharmaceutical compositions comprising therapeutically effective amounts of mycophenolate sodium Form M5 and at least one pharmaceutically acceptable carrier.

### BRIEF DESCRIPTION OF THE FIGURES

Figure 1 is a characteristic X-ray powder diffraction pattern for monosodium mycophenolate form M1.
Figure 2 is a characteristic FT-IR spectrum of Monosodium Mycophenolate form M1.

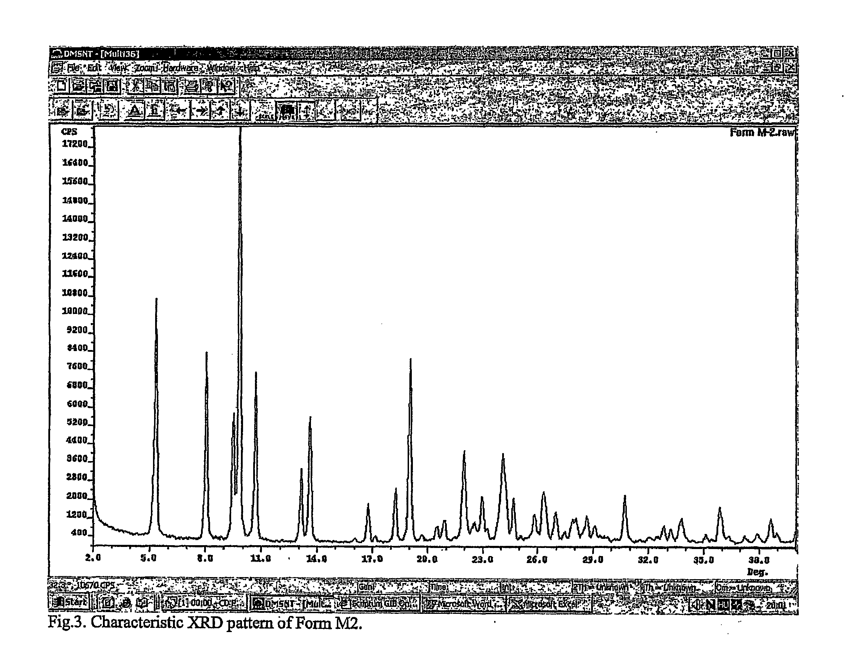

Figure 3 is a characteristic X-ray powder diffraction pattern for monosodium mycophenolate form M2.

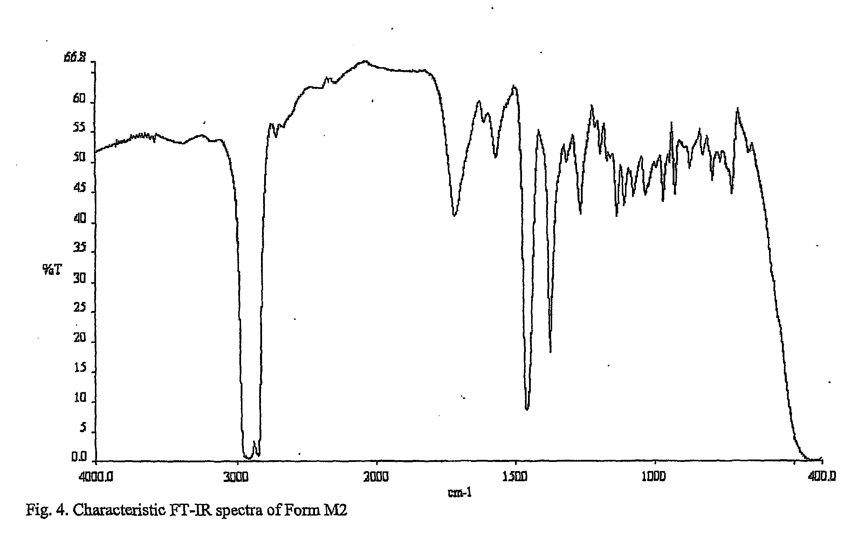

Figure 4 is a characteristic FT-IR spectrum of Monosodium Mycophenolate form M2.
Figure 5 is a characteristic X-ray powder diffraction pattern for monosodium mycophenolate form M3.
Figure 6 is a characteristic FT-IR spectrum of Monosodium Mycophenolate form M3.
Figure 7 is a characteristic X-ray powder diffraction pattern for monosodium mycophenolate form M5.
Figure 8 is a characteristic DSC curve for monosodium mycophenolate form M1.

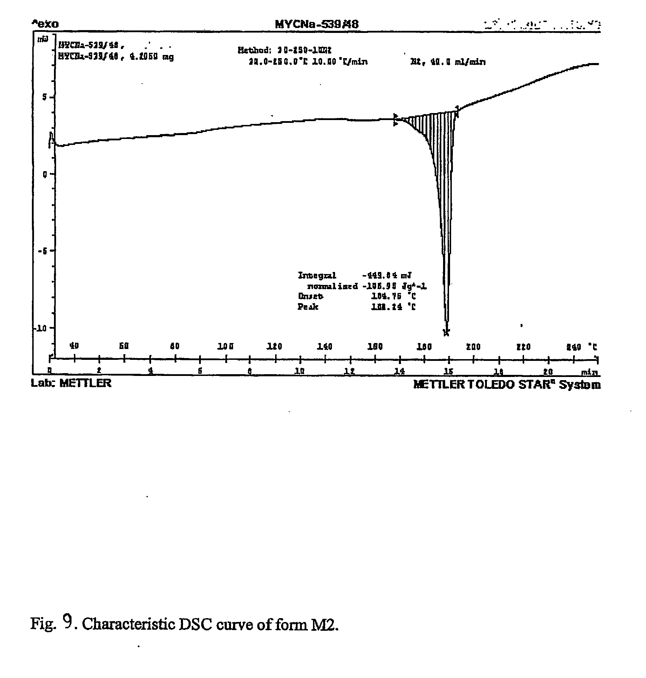

Figure 9 is a characteristic DSC curve for monosodium mycophenolate form M2.
Figure 10 is a characteristic DSC curve for monosodium mycophenolate form M3.
Figure 11 is a characteristic DSC curve for monosodium mycophenolate form M5.

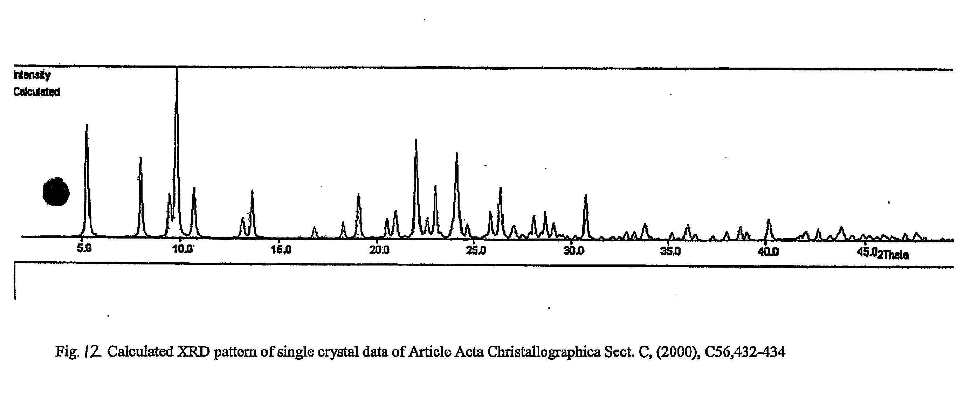

Figure 12 is a calculated XRD pattern of a single crystal data of Article Acta Crystallographica Sect. C, (2000), C56, 432-434.

### DETAILED DESCRIPTION OF THE INVENTION

The present invention provides a crystalline forms of monosodium mycophenolate denominated form and preparations thereof. The invention also encompasses pharmaceutical compositions comprising the mycophenolate forms of the invention.

The anhydrous mycophenolate sodium of this invention may be characterized by having not more than 1% weight loss. Mycophenolate sodium Form M5 discussed below is anhydrous mycophenolate sodium.

### Form M1

Crystalline mycophenolate sodium Form M1 is characterized by at least one of a powder XRD pattern with peaks at 4.7, 6.6, 11.2 and 15.6 ±0.2 degrees 2 theta (Fig. 1) or FTIR peaks at 3450, 1723, 1619, 1578, 1268 and 1132 cm-1 (Fig. 2). Form M1 may be further characterized by XRD peaks at 8.2, 21.5 and 23.4±0.2 degrees 2 theta. Form M1 may be further characterized by IR peaks at 1192, 1106, 1077, 1030, 971, 950, 921, 875, 794, 761, 723, and 666 cm-1. Form M1 is a hydrate having about 3.5 to about 5.0% water measured by TGA and Kar1-Fischer. The DSC melting peak of form M1 is at about 190-191°C (Fig. 8). Form M1 may be substantially free of other crystalline forms of mycophenolate sodium. The phrase "substantially free of other crystalline forms", as used herein, means that other crystalline forms make up less than or equal to about 10% by weight of the sample. Form M1 is a monohydrate.

One process for preparing crystalline mycophenolate sodium M1 includes preparing a solution of mycophenolic acid in a C₁-C₄ alcohol; combining a base and a source of sodium with the solution; cooling the mixture; and recovering the crystalline form. Recovery is preferably carried out within about 1 to 5 hours in that additional time may cause a transformation to another form. The process for preparing mycophenolate sodium Form M1 includes dissolving MPA in an alcohol, preferably methanol, preferably with stirring; adding a base and a source of sodium to the stirred reaction mixture dropwise at about room temperature; cooling the reaction mixture to about - 15°C; letting the reaction mixture stand at about -15°C, preferably for about 3 hours; and drying the isolated product, preferably at about 40-45°C in a vacuum oven.

As exemplified, to a stirred solution of MPA in methanol, preferably at a concentration of about 5 to about 10 ml/g, a source of sodium is added. Preferably, a 30% solution of sodium methoxide in methanol is added, more preferably dropwise at room temperature. The reaction mixture is then preferably cooled, more preferably to a temperature below about 0°C, most preferably to about - 15°C. Preferably, the cooling is carried out rapidly, within about 1 hour and then stirred for about 1-3 hours or until complete precipitation. The precipitated product is recovered and may be dried at about 40 to about 45°C in a vacuum oven.

Form M1 may also be prepared by drying a solution of mycophenolate sodium in water. The process for preparing mycophenolate sodium Form M1 includes dissolving sodium mycophenolate in water at elevated temperature, preferably about 60°C, preferably with stirring; allowing the solution to cool to about room temperature; and evaporating the solution to dryness. Drying may be carried out under ambient or reduced pressure.

Another process for preparing crystalline mycophenolate sodium Form M1 comprises preparing a solution of mycophenolic acid if a C3-C8 ketone; combining a base and a source of sodium with the solution to form a mixture; crystallizing the crystalline form from the mixture; and recovering the crystalline form. Form M1 is also prepared by crystallization from acetone, in presence of sodium ions, by addition of methanol.

Another process for preparing crystalline mycophenolate sodium M1 comprises preparing a solution of sodium mycophenolate in ethanol, preferably absolute ethanol; crystallizing the crystalline form from the solution; and recovering the crystalline form. Preferably the solution of step is heated, preferably to a temperature of at least about 60°C, and more preferably at to about reflux temperature. The solution is preferably cooled, more preferably at about room temperature, and allowed to crystallize over night

Another process for preparing crystalline mycophenolate sodium M1 comprises contacting crystalline mycophenolate sodium with water vapor. The crystalline mycophenolate sodium forms is preferably Form M3. Preferably, the crystalline mycophenolate sodium is placed in a hygroscopic chamber at about 100% relative humidity, preferably at about room temperature, and preferably for about one week or until Form M1 is obtained.

### Form M3

Crystalline mycophenolate sodium Form M3 is characterized by a powder XRD pattern with peaks at 6.0, 9.3, 15.5, and 18.4 ±0.2 degrees 2 theta (Fig. 5) and FTIR peaks at 3414,1713,1618,1567,1264 and 1134 cm-1 (Fig. 6). Form M3 may be further characterized by XRD peaks at 7.2, 10.8, 13.8, and 24.0 ±0.2 degrees 2 theta. Form M3 may be further characterized by IR peaks at 1415, 1312, 1202, 1108, 1078, 1034, 972, 947, 922, 877, 827, 809, 793, and 722 cm⁻¹. Form M3 may be substantially free of other crystalline forms of Mycophenolate sodium. Form M3 has a melting point at about 157 to about 158°C followed by an exothermic solid-solid transition, caused by the transformation of form M3 to form M2, which shows a DSC melting peak at about 188 to about 190 °C (Fig. 10). Weight loss of mass of heating up to melting is 0.7 % as measured by TGA. (0.8% by KF). Form M3 is anhydrous.

Form M3 is generally obtained by crystallization from a C₁ to C₄ alcohol, preferably methanol, followed by extended stirring. Form M3 has been obtained after leaving the crystals for extended, more than about 15 hours, more preferably about 20 to about 25 hours in the reaction mixture without stirring, or with presence of sodium hydroxide or sodium ethoxide in the absence of cooling. The extended stirring in the reaction mixture points to a transition of other forms to Form M3 in methanol overtime. As exemplified, the process includes (a) dissolving MPA in methanol with stirring, (b) adding a base and a source of sodium to the stirred reaction mixture dropwise at room temperature, (c) cooling the reaction mixture to -15°C over a period of 1 hour, (d) stirring the reaction mixture at -15°C for an additional 20-24 hours, (e) filtering off the precipitated product, (f) washing the isolated product with methanol, and (g) drying the isolated product at 40-45°C in a vacuum oven.

### Form M5

The present invention is a crystalline mycophenolate sodium, denominated Form M5, characterized by a powder XRD pattern with peaks at 9.8, 17.4, 22.2, 27.1, and 31.7 ±0.2 degrees 2 theta (Fig. 7). Form M5 may be further characterized by XRD peaks at 21.0, 26.3 and 31.4±0.2 degrees 2 theta. Form M5 may be substantially free of other crystalline forms of Mycophenolate sodium. Form M5 is an anhydrous form showing one DSC small endothermic peak in the range of 169-171 °C and a DSC melting peak in the temperature range of about 205 to about 221°C (Fig. 11). Weight loss is 0.8 % as measured by TGA. Form M5 is anhydrous.

The invention provides a process for preparing mycophenolate sodium Form M5. Form M5 may be obtained by from 1,4-dioxane. The process involves heating a mixture of sodium mycophenolate and 1,4-dioxane to obtain a solution, preferably to about 80°C or at reflux temperature, followed by crystallization of the crystalline form from the mixture, preferably by cooling the solution to room temperature. Finally, the crystalline form is recovered.

Preferably, the ratio of 1,4-dioxane to sodium mycophenolate is more than about 100 ml/g.

### Preparation of polymorphs by formation of sodium mycophenolate

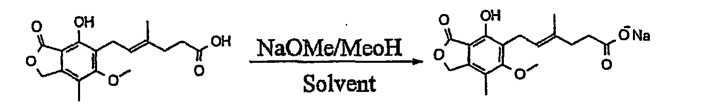

Sodium mycophenolate was obtained when MPA was reacted with methanolic NaOMe in different solvents and the product was crystallized. Form M1 or M2 or M3 were obtained.

### Preparation of polymorphs by recrystallization of sodium mycophenolate

Polymorphic purity: The crystal forms in the patent contain no more than about 5% of any other crystal forms.

The single particle size measured by polarizing light microscope of crystal forms described in the invention is less than about 100 micrometers.

The hygroscopicity of monosodium mycophenolate crystal forms M1, M2 and M3 were also investigated. Form M1, M2 and M3 were exposed to different level of humidity for one week and after equilibrium they were analysed by TGA and XRD for water content and crystal structure. Table 1 summarizes the results:

**Table 1.**

| **Crystal forms** | **Results** | | |
|---|---|---|---|
| | **%RH** | **LOD(%)** | **Form** |
| M1 | 20 | 4,5 | M1 |
| | 40 | 4,9 | M1 |
| | 60 | 5,1 | M1 |
| | 80 | 5,2 | M1 |
| | 100 | 31,2 | M1 |
| M2 | 40 | 0,1 | M2 |
| | 60 | 0,2 | M2 |
| | 80 | 0,4 | M2 |
| | 100 | 45,2 | M1 |
| M3 | 40 | 1,5 | M3>>M1 |
| | 60 | 2,3 | M3+M1 |
| | 80 | 4,9 | M1 |
| | 100 | 34,3 | M1 |

It can be established from the results that among the forms of monosodium salts, the most stable form at room temperature, at high relative humidity is the monohydrate form M1.

The equilibrium water content of M1 is about 5%. All tested monosodium crystal forms transform to M1 when achieving this water content.

In case of all tested samples, a very high level of water content were measured at 100% relative humidity.

Representatives of monosodium mycophenolate crystal forms, M1 and M2, were heated in an oven at atmospheric pressure at 130°C and 170°C for 30 minutes. The samples were analyzed by XRD before and after heating. Results are summarized in Table 2.

**Table 2: Crystal forms transformation by heating**

| **Crystal form before heating (XRD)** | **Crystal form after heating (XRD)** | |
|---|---|---|
| | **130 °C, 30 min.** | **170 °C, 30min.** |
| M1 | M3>>M1 | No data |
| M2 | M2 | M2 |

It can be seen in the table that heating of the sodium mycophenolate samples causes mostly change in the crystal form, except for form M2 of Monosodium salt.

This form is the most stable form at high temperature.

The present invention polymorphic form preferably contains less than 30%, more preferably less than 20%, more preferably less than 10% of other polymorphic forms by weight. The single particle size for the polymorph of the present invention is less than about 100 micrometers, as measured by polarizing light microscope of crystal described in the invention

The starting material used for the processes of the present invention, unless otherwise specified, may be any crystalline or amorphous form of mycophenolate sodium or acid, including various solvates and hydrates.

The processes of the present invention may also be practiced as the last step of prior art processes that synthesize mycophenolate sodium.

The base and the source of sodium as used throughout this invention can be different, or they can be the same. For example, sodium methoxide, sodium ethoxide, or sodium hydroxide can be used as both the base and the source of sodium. The preferred base and source of sodium is sodium methoxide.

Many processes of the present invention involve crystallization out of a particular solvent. One skilled in the art would appreciate that the conditions concerning crystallization may be modified without affecting the form of the polymorph obtained. For example, when mixing mycophenolate sodium in a solvent to form a solution, warming of the mixture may be necessary to completely dissolve the starting material. If warming does not clarify the mixture, the mixture may be diluted or filtered. To filter, the hot mixture may be passed through paper, glass fiber or other membrane material, or a clarifying agent such as celite. Depending upon the equipment used and the concentration and temperature of the solution, the filtration apparatus may need to be preheated to avoid premature crystallization.

The conditions may also be changed to induce precipitation. A preferred way of inducing precipitation is to reduce the solubility of the solvent. The solubility of the solvent may be reduced, for example, by cooling the solvent or adding an anti-solvent.

In one embodiment, an anti-solvent is added to a solution to decrease its solubility for a particular compound, thus resulting in precipitation. Another way of accelerating crystallization is by seeding with a crystal of the product or scratching the inner surface of the crystallization vessel with a glass rod.

In addition to the active ingredient(s), the pharmaceutical compositions of the present invention may contain one or more excipients. Excipients are added to the composition for a variety of purposes.

Diluents increase the bulk of a solid pharmaceutical composition and may make a pharmaceutical dosage form containing the composition easier for the patient and care giver to handle. Diluents for solid compositions include, for example, microcrystalline cellulose (e.g. Avicel^{®}), microfine cellulose, lactose, starch, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Solid pharmaceutical compositions that are compacted into a dosage Form like a tablet may include excipients whose functions include helping to bind the active ingredient and other excipients together after compression. Binders for solid pharmaceutical compositions include acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The dissolution rate of a compacted solid pharmaceutical composition in the patient's stomach may be increased by the addition of a disintegrant to the composition. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

Glidants can be added to improve the flowability of non-compacted solid composition and improve the accuracy of dosing. Excipients that may function as glidants include colloidal silicon dixoide, magnesium trisilicate, powdered cellulose, starch, talc and tribasic calcium phosphate.

When a dosage Form such as a tablet is made by compaction of a powdered composition, the composition is subjected to pressure from a punch and dye. Some excipients and active ingredients have a tendency to adhere to the surfaces of the punch and dye, which can cause the product to have pitting and other surface irregularities. A lubricant can be added to the composition to reduce adhesion and ease release of the product Form the dye. Lubricants include magnesium stearate, calcium stearate, glyceryl monostearate, glyceryl palmitostearate, hydrogenated castor oil, hydrogenated vegetable oil, mineral oil, polyethylene glycol, sodium benzoate, sodium lauryl sulfate, sodium stearyl fumarate, stearic acid, talc and zinc stearate.

Flavoring agents and flavor enhancers make the dosage Form more palatable to the patient. Common flavoring agents and flavor enhancers for pharmaceutical products that may be included in the composition of the present invention include maltol, vanillin, ethyl vanillin, menthol, citric acid, fumaric acid, ethyl maltol, and tartaric acid.

Solid and liquid compositions may also be dyed using any pharmaceutically acceptable colorant to improve their appearance and/or facilitate patient identification of the product and unit dosage level.

In liquid pharmaceutical compositions of the present invention hydrochloride Forms and any other solid excipients are dissolved or suspended in a liquid carrier such as water, vegetable oil, alcohol, polyethylene glycol, propylene glycol or glycerin.

Liquid pharmaceutical compositions may contain emulsifying agents to disperse uniformly throughout the composition an active ingredient or other excipient that is not soluble in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid pharmaceutical compositions of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

Sweetening agents such as sorbitol, saccharin, sodium saccharin, sucrose, aspartame, fructose, mannitol and invert sugar may be added to improve the taste.
Preservatives and chelating agents such as alcohol, sodium benzoate, butylated hydroxy toluene, butylated hydroxyanisole and ethylenediamine tetraacetic acid may be added at levels safe for ingestion to improve storage stability.

A liquid composition according to the present invention may also contain a buffer such as guconic acid, lactic acid, citric acid or acetic acid, sodium guconate, sodium lactate, sodium citrate or sodium acetate.

Selection of excipients and the amounts to use may be readily determined by the Formulation scientist based upon experience and consideration of standard procedures and reference works in the field.

The solid compositions of the present invention include powders, granulates, aggregates and compacted compositions. The dosages include dosages suitable for oral, buccal, rectal, parenteral (including subcutaneous, intramuscular, and intravenous), inhalant and ophthalmic administration. Although the most suitable route in any given case will depend on the nature and severity of the condition being treated, the most preferred route of the present invention is oral. The dosages may be conveniently presented in unit dosage Form and prepared by any of the methods well-known in the pharmaceutical arts.

Dosage Forms include solid dosage Forms like tablets, powders, capsules, suppositories, sachets, troches and losenges as well as liquid syrups, suspensions and elixirs.

A dosage Form of the present invention is a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.
The active ingredient and excipients may be Formulated into compositions and dosage Forms according to methods known in the art.

A composition for tableting or capsule filing may be prepared by wet granulation. In wet granulation some or all of the active ingredients and excipients in powder Form are blended and then further mixed in the presence of a liquid, typically water, that causes the powders to clump up into granules. The granulate is screened and/or milled, dried and then screened and/or milled to the desired particle size. The granulate may then be tableted or other excipients may be added prior to tableting such as a glidant and or lubricant.

A tableting composition may be prepared conventionally by dry blending. For instance, the blended composition of the actives and excipients may be compacted into a slug or a sheet and then comminuted into compacted granules. The compacted granules may be compressed subsequently into a tablet.

As an alternative to dry granulation, a blended composition may be compressed directly into a compacted dosage Form using direct compression techniques. Direct compression produces a more uniform tablet without granules. Excipients that are particularly well suited to direct compression tableting include microcrystalline cellulose, spray dried lactose, dicalcium phosphate dihydrate and colloidal silica. The proper use of these and other excipients in direct compression tableting is known to those in the art with experience and skill in particular Formulation challenges of direct compression tableting.

A capsule filling of the present invention may comprise any of the aforementioned blends and granulates that were described with reference to tableting, only they are not subjected to a final tableting step.

### Experimental methodology (physical)

### XRD

ARL X-ray powder diffractometer model X'TRA-030, Peltier detector, round standard aluminum sample holder with round zero background quartz plate was used. Scanning parameters: Range: 2-40 deg. 2 θ, continuous Scan, Rate: 3 deg./min. The accuracy of peak positions is defined as +/- 0.2 degrees due to experimental differences like instrumentations, sample preparations etc.

### FTIR Spectroscopy

Perkin-Elmer Spectrum 1000 Spectrometer, at 4 cm⁻¹ resolution with 16 scans, in the range of 4000-400 cm⁻¹ was used. The samples were analyzed in Nujol mull. The spectra were recorded using an empty cell as a background.

### Differential Scanning Calorimetry (DSC)

DSC 822^{e}/700, Mettler Toledo, Sample weight: 3-5 mg.
Heating rate: 10 °C/min., Number of holes of the crucible: 3
In N₂ stream: flow rate = 40 ml/min
Scan range: 30-250°C, 10°C/ minutes heating rate.

The DSC curves of the novel forms of mycophenolate monosodium indicates only endothermic peaks due to dehydration, desolvation and melting.

### Thermal Gravimetric Analysis (TGA)

TGA/SDTA 851^{e}, Mettler Toledo, Sample weight 7-15 mg.
Heating rate: 10 °C/ min., In N₂ stream: flow rate = 50 ml/min
Scan range: 30-250°C.

### Microscope

The particle size of single crystals was observed by a polarizing light Microscope, Type : Zeiss TOPIC-B. Sample preparation was done by dispersing a sample in one drop of mineral oil. The magnification was 200.

### Abbreviations

MPA = Mycophenolic acid
NaOMe = Sodium methoxide
DMF = Dimethyl formamide

### Preparation of mycophenolate monosodium crystal forms

### Example 1 (Reference)

To a stirred solution of MPA (6.4 g) in methanol (32 ml), 30% sodium methoxide in methanol (3.8 ml) was added dropwise at room temperature. The reaction mixture was stored at -15 °C. After 3 hours, the precipitated product was filtered and then washed with cold methanol. The solid material was dried at 40-45 °C in a vacuum oven. Form M1 of mycophenolate sodium was obtained in 48% yield.

### Example 2 (Reference)

To a stirred solution of MPA (6.4 g) in methanol (64 ml), 30% sodium methoxide in methanol (3.8 ml) was added dropwise at room temperature. The reaction mixture was cooled to -15 °C within 1 hour and stirred at this temperature for an additional 2 hours. The precipitated product was filtered and then washed with cold methanol. The solid material was dried at 40-45°C in a vacuum oven. Form M1 of mycophenolate sodium was obtained in 54% yield.

### Example 3 (Reference)

To a stirred solution of MPA (6.4 g) in methanol (32 ml), 30% sodium methoxide in methanol (4.5 ml) was added dropwise at room temperature. After stirring at room temperature for 0.5 h, the reaction mixture was stored at -15°C. After 1.5 hours, the precipitated product was filtered off and washed with cold methanol. The solid material was dried at 40-45°C in a vacuum oven. Form M1 of mycophenolate sodium was obtained in 43% yield.

### Example 4 (Reference)

To a stirred solution of MPA (6.4 g) in methanol (64 ml) 30% of sodium methoxide in methanol (3.8 ml) was added dropwise at room temperature. The reaction mixture was cooled to -15 °C within 1 hour and stirred at this temperature for additional 20 hours. The precipitated product was filtered, then washed with cold methanol. A part of the solid material was dried at 40-45 °C in vacuum oven. The yield was 40%. Form M3 of mycophenolate sodium was obtained from the dry sample.

### Example 5

Sodium mycophenolate (1 g) was heated to reflux temperature in 1,4-dioxane (400 ml). The salt was not dissolved completely. The mixture was allowed to cool to room temperature, and crystallized at this temperature overnight. The solid was filtered off, and a part of the wet material was dried at normal pressure at room temperature. Form M5 of mycophenolate sodium was obtained from both the wet sample and the dry sample. Water content of the dry sample was 1.8 % measured by KF.

### Example 6 (Reference)

Sodium mycophenolate (1 g) was dissolved at reflux temperature in absolute ethanol (165 ml). The solution was allowed to cool to room temperature, and crystallized at this temperature overnight. The solid was filtered off, and a part of the wet material was dried at normal pressure at room temperature. Form M1 of mycophenolate sodium was obtained from the wet sample. Form M2 of mycophenolate sodium was obtained from the dry sample.

### Example 7 (Reference)

Sodium mycophenolate (1 g) was dissolved at reflux temperature in 1-butanol (175 ml). The solution was allowed to cool to room temperature, and crystallized at this temperature overnight. The solid was filtered off, and a part of the wet material was dried at normal pressure at room temperature. Form M2 of mycophenolate sodium was obtained from both the dry and the wet sample.

### Example 8 (Reference)

Sodium mycophenolate (1 g) was dissolved at about 60 °C in water (5 ml). The solution was allowed to cool to room temperature. The solution was then allowed to evaporate to dryness at room temperature in an open dish. Form M1 of mycophenolate sodium was obtained

### Example 9 (Reference)

200 mg of each of the following samples: crystal Form M2 and crystal Form M3 1 were put at room temperature into a hygroscopicity chamber containing 100% relative humidity for one week. After one week they were measured again by XRD to determine their crystal forms. Crystal forms M2 and M3 transformed to M1.

### Reproduction of literature methods

### Example 10 (Acta Cryst. Sect. C, C56 (2000) 432-433)

To a stirred solution of MPA (9.6 g) in methanol (300 ml), 30% sodium methoxide in methanol (5.6 ml) was added dropwise at room temperature. The reaction mixture was stirred for an additional 60 minutes, then the solvent was evaporated on a rotary evaporator at 40-45°C under vacuum. The wet material was dried at 40-45°C in a vacuum oven and proved to be a mixture of Forms M2 and M3.

### Example 11 (J. Med. Chem., 39 (1996) 1236-1242)

To a stirred solution of MPA (9.6 g) in absolute ethanol (360 ml), 21% sodium ethoxide in ethanol (8.6 ml) was added dropwise at room temperature. The reaction mixture was stirred for an additional 60 minutes, then the solvent was evaporated on a rotary evaporator at 40-45°C under vacuum. The wet material was dried at 40-45°C in a vacuum oven and proved to be Form M2.

### Example 12 (ZA 68/4,959)

To a stirred solution of MPA (13 g) in chloroform (650 ml), sodium methoxide solution (2.3 g NaOMe dissolved in 130 ml of methanol) was added dropwise at room temperature. The reaction mixture was stirred for an additional 30 minutes, then n-pentane (2.34 L) was added to the solution. After 30 minutes, the reaction mixture was filtered and a part of the wet material was dried at 40-45°C in a vacuum oven. Both the wet sample and the dried material proved to be Form M2.

## Claims

1. Anhydrous crystalline mycophenolate sodium form (Form M5) **characterized by** a powder XRD pattern with peaks at 9.8,17.4, 22.2, 27.1, and 31.7 ±0.2 degrees 2-theta.

2. Anhydrous crystalline mycophenolate sodium according to Claim 1 further **characterized by** XRD peaks at 21.0, 26.3, and 31.4 ± 0.2 degrees 2-theta.

3. Anhydrous crystalline mycophenolate sodium according to Claim 1 or Claim 2 **characterized by** an XRD as depicted in Figure 7.

4. Anhydrous crystalline mycophenolate sodium according to any preceding claim showing one DSC small endothermic peak in the range of 169-171°C and a DSC melting peak in the temperature range of about 205 to about 221 °C.

5. Anhydrous crystalline mycophenolate sodium according to Claim 4 **characterized by** a DSC as depicted in Figure 11.

6. Anhydrous crystalline mycophenolate sodium according to any preceding claim having a weight loss of 0.8% as measured by TGA.

7. Anhydrous crystalline mycophenolate sodium according to any preceding claim containing less than or equal to about 10% by weight of other crystalline forms of mycophenolate sodium.

8. A process for preparing the anhydrous crystalline mycophenolate sodium of any preceding claim comprising heating a mixture of sodium mycophenolate and 1,4-dioxane to obtain a solution, followed by crystallization of the crystalline form from the mixture and recovering the crystalline form.

9. A process according to Claim 8 wherein the mixture is heated to about 80°C or at reflux temperature.

10. A process according to Claim 8 or Claim 9 wherein the crystallisation is carried out by cooling the solution to room temperature.

11. A process according to any of Claims 8-10 wherein the ratio of 1,4-dioxane to sodium mycophenolate is more than about 100 ml/g.

12. A pharmaceutical composition comprising a therapeutically effective amount of a mycophenolate sodium form as defined in any of Claims 1-7, and at least one pharmaceutically acceptable carrier.

## Patentansprüche

1. Form von wasserfreiem kristallinen Mycophenolat-Natrium (Form M5), **gekennzeichnet durch** ein XRD Pulverdiagramm mit Scheitelwerten bei 9,8, 17,4, 22,2, 27,1 und 31,7 ± 0,2 Grad 2-Theta.

2. Wasserfreies kristallines Mycophenolat-Natrium gemäß Anspruch 1, welches weiter durch XRD-Scheitelwerte bei 21,0, 26,3 und 31,4 ± 0,2 Grad 2-Theta **gekennzeichnet** ist.

3. Wasserfreies kristallines Mycophenolat-Natrium gemäß Anspruch 1 oder Anspruch 2, welches durch ein XRD wie in Figur 7 dargestellt, **gekennzeichnet** ist.

4. Wasserfreies kristallines Mycophenolat-Natrium gemäß einem der vorangehenden Ansprüche, welches einen kleinen endothermen DSC-Scheitelwert im Bereich von 169-171 °C und einen DSC-Schmelzscheitelwert in dem Temperaturbereich von ungefähr 205 bis ungefähr 221 °C aufweist.

5. Wasserfreies kristallines Mycophenolat-Natrium gemäß Anspruch 4, welches durch ein DSC, wie in Figur 11 dargestellt, **gekennzeichnet** ist.

6. Wasserfreies kristallines Mycophenolat-Natrium gemäß einem der vorangehenden Ansprüche, welches einen mit TGA gemessenen Gewichtsverlust von 0,8% aufweist.

7. Wasserfreies kristallines Mycophenolat-Natrium gemäß einem der vorangehenden Ansprüche, welches weniger als oder in etwa 10 Gew.-% anderer kristalliner Formen von Mycophenolat-Natrium enthält.

8. Verfahren zur Herstellung von wasserfreiem kristallinen Mycophenolat-Natrium nach einem der vorangehenden Ansprüchen, welches Erhitzen eines Gemischs aus Natriummycophenolat und 1,4-Dioxan unter Erhalt einer Lösung gefolgt von Kristallisieren der kristallinen Form aus dem Gemisch und Wiedergewinnen der kristallinen Form umfasst.

9. Verfahren gemäß Anspruch 8, wobei das Gemisch auf ungefähr 80°C oder auf Rückflusstemperatur erhitzt wird.

10. Verfahren gemäß Anspruch 8 oder Anspruch 9, wobei das Kristallisieren durch Kühlen der Lösung auf Raumtemperatur durchgeführt wird.

11. Verfahren gemäß einem der Ansprüche 8-10, wobei das Verhältnis von 1,4-Dioxan zu Natriummycophenolat mehr als ungefähr 100 ml/g ist.

12. Pharmazeutische Zusammensetzung, welche eine therapeutisch wirksame Menge an einer Form von Mycophenolat-Natrium, wie in einem der Ansprüche 1-7 definiert, und wenigstens einen pharmazeutisch verträglichen Trägerstoff umfasst.

## Revendications

1. Forme cristalline anhydre du mycophénolate de sodium (forme M5) **caractérisée par** un diagramme de diffraction des rayons X de poudre avec des pics à 9,8, 17,4, 22,2, 27,1 et 31,7 ± 0,2 degrés 2-thêta.

2. Mycophénolate de sodium cristallin anhydre selon la revendication 1 **caractérisé en outre par** des pics de diffraction des rayons X à 21,0, 26,3 et 31,4 ± 0,2 degrés 2-thêta.

3. Mycophénolate de sodium cristallin anhydre selon la revendication 1 ou la revendication 2 **caractérisé par** un diagramme de diffraction des rayons X tel que représenté sur la figure 7.

4. Mycophénolate de sodium cristallin anhydre selon l'une quelconque des revendications précédentes présentant un petit pic endothermique en CDB dans la plage de 169-171°C et un pic de fusion en CDB dans la plage de température d'environ 205 à environ 221 °C.

5. Mycophénolate de sodium cristallin anhydre selon la revendication 4 **caractérisé par** une CDB telle que représentée sur la figure 11.

6. Mycophénolate de sodium cristallin anhydre selon l'une quelconque des revendications précédentes ayant une perte de poids de 0,8 % telle qu'elle est mesurée par ATG.

7. Mycophénolate de sodium cristallin anhydre selon l'une quelconque des revendications précédentes contenant moins d'environ 10 % en poids ou environ 10 % en poids d'autres formes cristallines du mycophénolate de sodium.

8. Procédé pour préparer le mycophénolate de sodium cristallin anhydre selon l'une quelconque des revendications précédentes comprenant le chauffage d'un mélange de mycophénolate de sodium et de 1,4-dioxane pour obtenir une solution, suivi par la cristallisation de la forme cristalline dans le mélange et la récupération de la forme cristalline.

9. Procédé selon la revendication 8 où le mélange est chauffé à environ 80°C ou à la température de reflux.

10. Procédé selon la revendication 8 ou la revendication 9 où la cristallisation est réalisée par refroidissement de la solution à la température ambiante.

11. Procédé selon l'une quelconque des revendications 8-10 où le rapport du 1,4-dioxane au mycophénolate de sodium est supérieur à environ 100 ml/g.

12. Composition pharmaceutique comprenant une quantité thérapeutiquement efficace d'une forme de mycophénolate de sodium telle que définie dans l'une quelconque des revendications 1-7 et au moins un vecteur pharmaceutiquement acceptable.
